# EUROPEAN PATENT APPLICATION

(11) **EP 2 818 194 A1**
(43) Date of publication of application: **31.12.2014**
(21) Application number: 13173923.7
(22) Date of filing: 27.06.2013
(51) Int. Cl.: A61M 16/06

(54) **Nasal respiratory mask integrally made of silicone**

(71) Applicant: Air Liquide Medical Systems, 92160 Antony (FR)
(72) Inventor: Alberici, Luca, 25128 Brescia (IT); Masserdotti, Fulvio, 25075 Brescia (IT); Sandoni, Giuseppe, 25124 Brescia (IT)
(74) Representative: Pittis, Olivier

(57) **Abstract**

A respiratory mask, especially a nasal mask for patients having variable facial morphologies, comprising a mask body (1) comprising an internal chamber (6) and an inlet orifice (4) in fluid communication with the internal chamber, a holding arm (3) arranged on the mask body and projecting upwardly from said mask body, a cushion (5) having an aperture (7) adapted for receiving at least part of the patient's nose, when the patient wears the mask, and two lateral arms (2a, 2b) arranged on each side of the mask body and projecting laterally from said mask body. Further, the mask body (1), the holding arm (3), the cushion (5) and the two lateral arms (2a, 2b) are integral and form a single piece made of a resilient soft material. The mask of the invention is preferably a pediatric nasal mask for use especially with children, infants, toddlers or newborns.

## Description

The invention concerns a respiratory mask, preferably a nasal mask, in particular a pediatric nasal mask, for use in the treatment of respiratory conditions or diseases, especially affecting infants, children or the like, whatever their facial morphology.

Nasal masks are commonly used for delivering non-invasive positive pressure ventilation (NPPV) or for nasal continuous positive airway pressure (N-CPAP) therapy in disordered breathing conditions, such as obstructive sleep apnea (OSA), chronic obstructive pulmonary disease (COPD)...

Nasal masks deliver a flow of breathable gas for, or to assist in, patient respiration. Such a mask assembly typically comprises a rigid or semi-rigid hollow shell, usually made of polymer, defining a breathing chamber that receives at least a part of the patient's nose and further comprising a soft face-contacting cushion that comes into contact with the patient's face and conforms to the various facial contours of the patient face, which cushion is usually made of soft, resilient elastomeric material, such as soft silicone or similar, and a forehead support and a headgear for correctly positioning, maintaining and/or securing the mask on the head of a patient.

The forehead support is usually arranged on an expansion part of the mask, i.e. a portion of the mask body forming a holding arm that projects upwardly from the mask body and in the direction of the forehead of the user when the mask is positioned on the user's face, which holding arm is also usually made of polymer. The hollow shell typically receives a gas supply line which delivers a respiratory gas, such as air under pressure, into the breathing chamber of the shell. An example of a nasal mask of this kind is given by EP-A-462701.

The mask assembly is secured to the wearer's head by straps or similar devices thereby forming a headgear that can be adjusted to pull the mask against the face with sufficient force to achieve a gas tight seal between the mask and the wearer's face as taught by documents EP-A-462701, EP-A-874667, EP-A-1972357 or WO-A-00/57942.

However, with the current masks, it is often difficult to obtain an efficient positioning of the mask on the patient's face and to ensure sufficient tightness (seal) preventing the escape of gas as well as a comfort of use for the patient, when the patient is an infant, such as a newborn, a baby, a toddler or a child.

Document EP-A-2114500 proposes a nasal mask useable with infants and children, which includes a flexible cushion with a tube connection portion at one or both sides adjacent the patient's nares thereby allowing the patient to sleep on their face more comfortably. A more rigid support structure adjacent the cushion and a headgear are provided to stabilize the cushion and prevent it from collapsing.

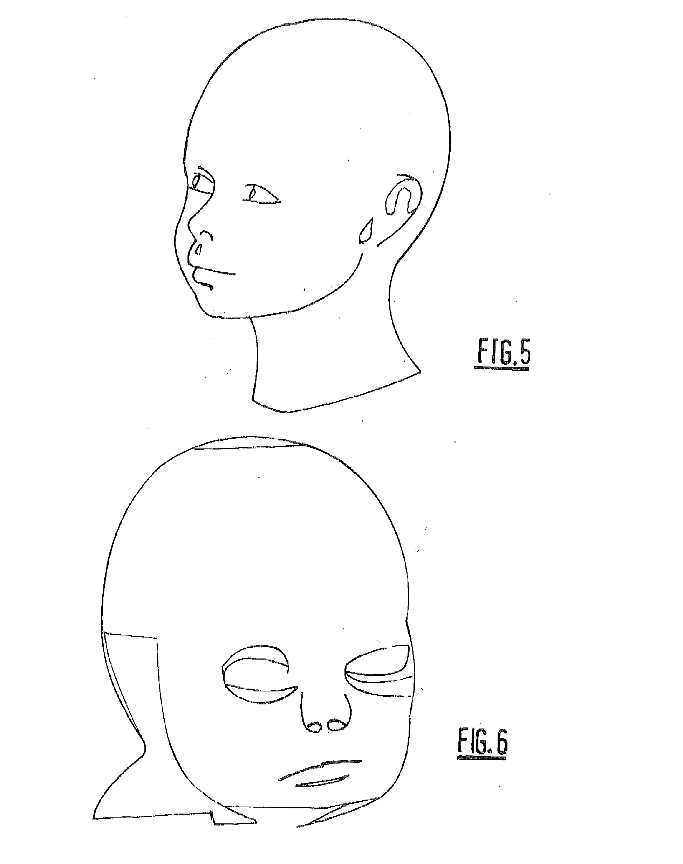

This document does not take into account the very variable morphology of children and infants. In other words, today existing pediatric masks, especially nasal masks, are not entirely satisfying and need to be improved to better account for the very variable morphology of children and infants as illustrated in Figures 5 and 6.

Indeed, as the morphology of the face of infants and children is different from one user to another and change very quickly while they are growing up, it is important to be able to correctly position the mask on the user's nasal region and afterwards maintain it in a desired position, whatever the morphology of the patient is, so as to ensure efficient gas tightness (seal), increased comfort and/or avoid the possibility to have face deformations due to change of the morphology on infants, toddlers and children.

Hence, the problem to be solved is to provide an improved mask architecture ensuring efficient gas tightness (seal) and/or increased comfort for the user or patient, especially when said user or patient is an infant, a toddler or a child.

The solution of the present invention concerns a respiratory mask, in particular a nasal mask for pediatric use, comprising:
- a mask body comprising an internal chamber and an inlet orifice in fluid communication with the internal chamber,
- a holding arm arranged on the mask body and projecting upwardly from said mask body,
- a cushion having an aperture for receiving at least part of the patient's nose, when the patient wears the mask, and
- two lateral arms arranged on each side, i.e. right and left sides, of the mask body and projecting laterally from said mask body,
**characterized in that** the mask body, the holding arm, the cushion and the two lateral arms are integral and form a single piece made of a resilient soft material.

The mask according to the present invention can further comprise one or more of the following additional features:
- the mask body, the holding arm, the cushion and the two lateral arms are molded in one piece.
- one of the two lateral arms projects from the right side of the mask body, while the other of the two lateral arms projects from the left side of the mask body.
- the resilient soft material forming the single piece is made of silicone or the like.
- the cushion comprises at least one flexible membrane, said membrane being made of said resilient soft material and is integral with the cushion.
- an annular element is arranged in the inlet orifice of the mask body, preferably an annular element made of a rigid polymer, such as plastic.
- the holding arm projecting upwardly from said mask body comprises at least one slot at its terminal end.
- at least one of the two opposite lateral arms projecting laterally from said mask body comprises a slot.
- the respiratory mask further comprises a headgear and connecting elements for fixing the headgear to the mask body.
- the connecting elements comprise one or several straps, wherein the one or several straps of the headgear cooperate with one of the slots arranged at the terminal end of the holding arm and/or in at least one of the two lateral arms.
- the mask body has a generally triangular tridimensional shape.
- the inlet orifice is arranged at the center of the mask body.
- the opposite lateral arms projecting laterally from said mask body comprise connecting structures for fixing a headgear.
- one of the opposite lateral arms projecting laterally from said mask body comprises a slot or hole for fixing a strap of a headgear, preferably the left arm.
- one of the opposite lateral arms projecting laterally from said mask body, preferably the right arm, comprises a hollow connecting structure 14 having a tridimensional shape, such as a semi-cylindrical shape, projecting away on the front surface of said lateral arm and the hollow connecting structure 14 further comprises an inner passage configured for receiving the hook of a hook structure that further comprises a rear buckle or loop for fixing thereto a strap of a headgear, thereby allowing an easy connection of the headgear to the mask body, and a quick release in case of emergency.
- the two opposite lateral arms have a generally curved-shape, preferably the two lateral arms are curved toward the rear and bottom side of the mask body.
- the respiratory mask is a nasal mask.
- the respiratory mask is pediatric nasal mask dimensioned for fitting to the nasal region of an infant, a child or similarly small patients, i.e. including toddlers, newborns....
- the cushion comprises an aperture having a triangular form or other similar shapes.

The invention also concerns an assembly comprising a gas delivery device, such as a medical ventilator, and a respiratory mask according to the present invention, preferably the gas delivery device is connected to the respiratory mask by means of a gas line, such as a flexible hose.

A preferred embodiment of a nasal mask according to the present invention is shown in the enclosed Figures, among which :
- Figure 1 represents a front view of an embodiment of a nasal mask according to the present invention for pediatric usage,
- Figure 2 is a lateral view of the mask of Figure 1,
- Figure 3 is a rear view of the mask of Figure 1,
- Figure 4 shows the tubing elements fixed to the mask of Figure 1,
- Figure 5 shows an example of the facial morphology of a child,
- Figure 6 shows an example of the facial morphology of an infant/baby,
- Figure 7 shows a hook structure fixed to the mask of Figure 1,
- Figure 8 shows an enlarged view of the hook structure of Figure 7, and
- Figure 9 is an enlarged view from above of the right arm of the mask of Figures 1 to 4, 7 and 8.

As illustrated in Figures 5 and 6, the facial morphologies of children or toddlers of one or several years (Fig. 5) and of infants/babies of several days, weeks or months (Fig. 6) are very dissimilar, i.e. very different, and they change very quickly while the infant/child is growing up.

This becomes an important problem when the child/infant should receive a gaseous treatment delivered by means of a respiratory mask because the mask to be used should well-fit with any facial morphology in order to get an efficient gas delivery.

However, with the current masks, it is often difficult to obtain an efficient positioning of the mask on the patient's face and to ensure a sufficient tightness (seal) preventing the escape of gas as well as a good comfort of use for the patient, especially when the patient is an infant, such as a newborn or a baby, or a child/toddler. This may be also true for some adult patients.

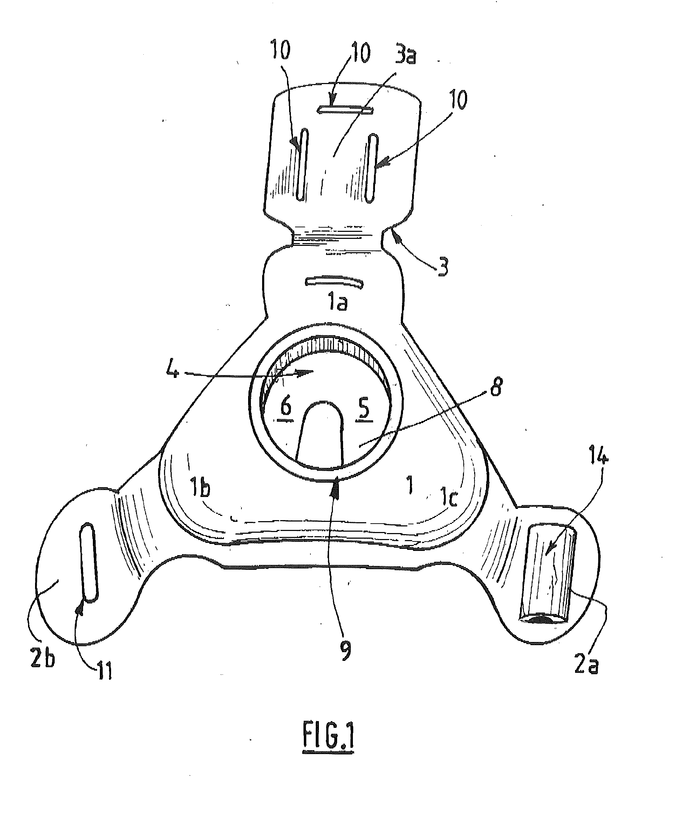

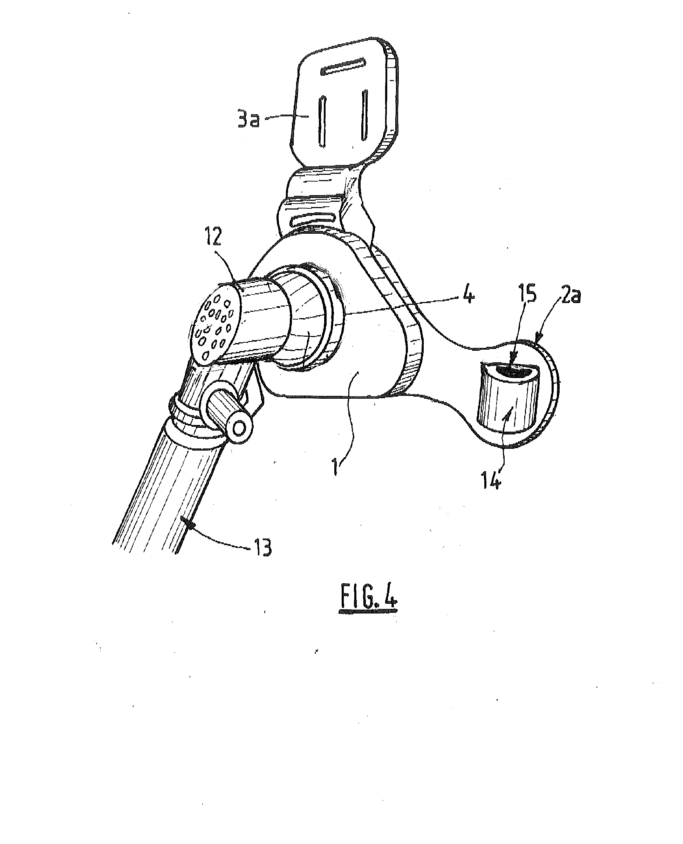

In order to overcome these problems, the present invention proposes a new type of respiratory nasal mask, such as a pediatric nasal mask, as illustrated in Figures 1 to 4 and 7 to 9.

Generally speaking, a respiratory mask according to the present invention comprises a hollow shell or mask body 1 defining an internal breathing chamber 6 or volume, wherein respiratory gas, such as air under pressure, is introduced via an inlet port 4 to which is connected a gas feeding line 13, such as a flexible hose, by means of a tubular connector 12 or similar.

The gas inlet orifice 4 is arranged at the center of the front side of the mask body 1 and through its wall, thereby allowing gas under pressure to be introduced in the breathing chamber 6, said inlet orifice 4 being in fluid communication with the internal chamber 6.

The mask body 1 has preferably a generally triangular tridimensional shape as visible in Figure 1 for example so as to better match the nasal regions of the patient.

Figures 1 to 6 illustrate an embodiment of a mask body 1 according to the present invention that is configured to a pediatric usage. The mask body 1 receives at least a part of the patient's nose, when said patient is an infant/baby or a child that introduces his/her nose into the internal volume of the breathing chamber 6 of the mask body 1 for breathing of the gas contained therein.

Further, the mask body 1 also comprises, on the one hand, a holding arm 3 forming an upper or frontal support that is integral with the mask body 1 and projects upwardly from said mask body 1 and, one the other hand, two lateral arms or wings 2a, 2b that are also integrally arranged on the mask body 1 and that project laterally, i.e. on each opposite lateral sides (right and left sides) from said mask body 1 so as to constitute right and left cheek supports, when the mask is worn by a patient, as illustrated in Figures 1-3.

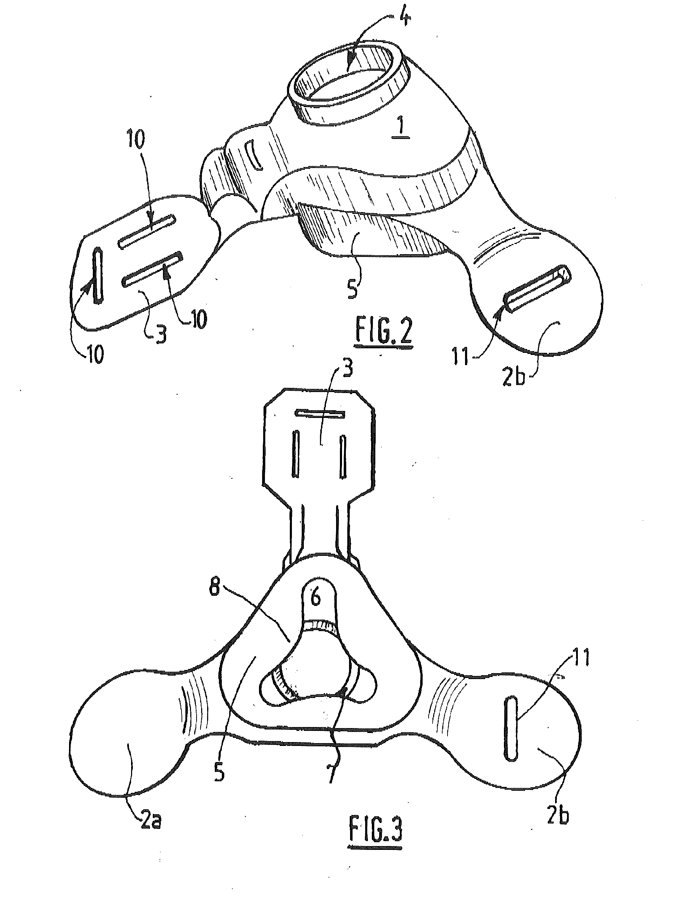

Furthermore, a cushion 5 having an aperture 7 for receiving at least part of the patient's nose, when the patient wears the mask, is arranged on the rear side of the mask body 1 as shown in Figure 3.

According to the present invention, in order to provide efficient gas tightness (seal) and/or increased comfort for the patient, especially when said patient is a infant or child, the mask body 1, the holding arm 3, the cushion 5 and the two lateral arms 2a, 2b are made of a single piece of a resilient soft material, such as preferably silicone. Preferably, they are molded in one piece.

Said single piece of resilient soft material being entirely flexible, it can be adapted to a great variety of facial morphologies, especially of various nasal regions of infant or child patients.

Using a soft material also avoids the risks that some infant/child patients receiving gas treatment delivered by respiratory masks, while they are growing up, encounter facial deformations due to an excessive rigidity of the mask.

Preferably, the soft resilient cushion 5 that comes into contact with the patient's face, during use of the mask, comprises a central aperture 7 for receiving at least a part of the patient's nose, which can have a triangular or similar shape or structure as illustrated in Figure 3, so as to match the contours of the nasal region of the patient.

A soft flexible membrane 8 forming a kind of skirt delimits said central aperture 7. Said soft flexible membrane 8 is molded in one piece with the rest of the cushion 5 so as to form part of the single piece of flexible material.

Preferably, the respiratory mask according to the present invention is a nasal mask. In that case, the cushion 5 and the membrane 8 comprise an upper nasal bridge region, a lower region and two lateral regions (i.e. left and right regions) connecting the nasal bridge and lower regions.

When the mask is worn by the patient, the upper nasal bridge region is in contact with the nasal bridge of the patient, the lower region is in contact with the area between the nose and the upper lip of the patient, and the lateral opposite regions are in contact with the flange regions of the nose of the patient.

Further, the cushion 5 can comprise one or several membranes 8, such as two superimposed membranes. Using several membranes may improve the gas tightness. Nevertheless, in the present embodiment, a unique membrane is provided.

Further, in the embodiment of Figure 1, the mask body 1 according to the present invention has a generally triangular tridimensional shape that more or less matches or corresponds to the general structure of a nose. Said generally triangular tridimensional shape or structure comprises three angle areas 1a-1c forming the three corners of the triangular structure of the mask body 1.

The holding arm 3 projects upwardly from one la of the three angle areas la-lc of said mask body 1, whereas the two lateral arms 2a, 2b project laterally from the two other angle areas 1b,1c of said mask body (1) as illustrated in Figure 1.

The holding arm 3 comprises one or several slots 10 or similar traversing orifices or holes of any shapes, preferably located at its terminal end 3a, for receiving and fixing one or several straps of a headgear (not shown)

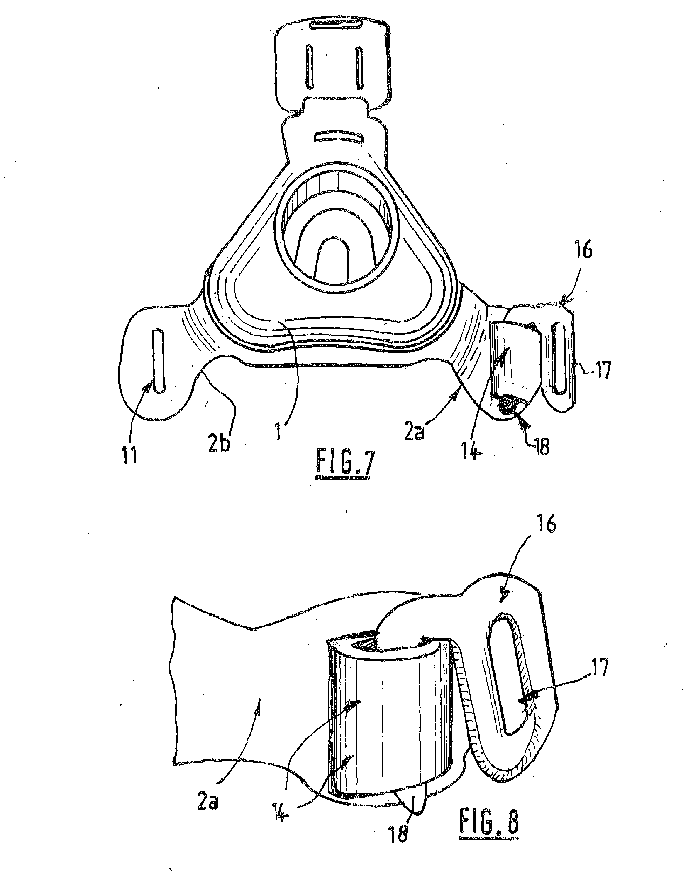

Further, in the present embodiment, the left arm 2b of the mask as illustrated in Figures 1 and 7 also comprises a slot 11 for receiving and fixing a strap of the headgear, whereas the right arm 2a of the mask comprises a hollow connecting structure 14 with a inner passage 15, said hollow connecting structure 14 projecting away on the front surface of the right arm 2a, preferably in the end region of the right arm 2a.

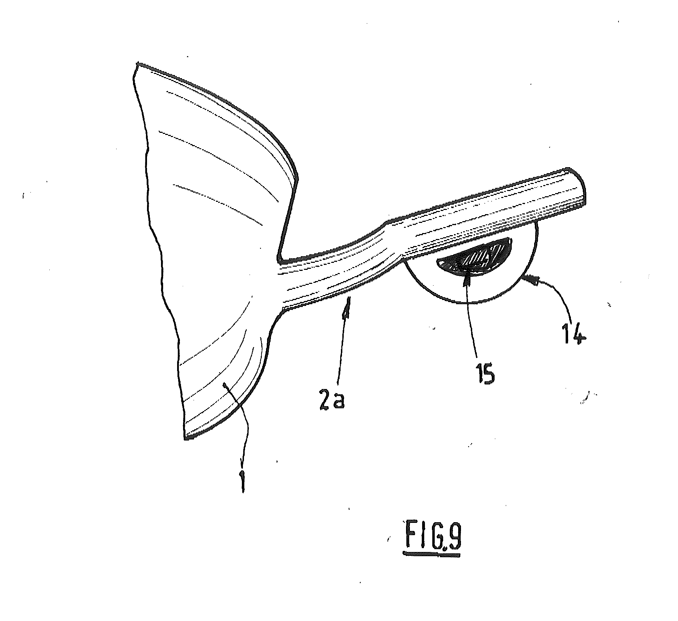

The hollow connecting structure 14 has a tridimensional shape, such as the semi-cylindrical shape illustrated in Figure 9.

This hollow connecting structure 14 and its inner passage 15 are configured for receiving the hook 18 of a hook structure 16 that is introduced into the inner passage 15 of the hollow connecting structure 14 as illustrated in Figures 7 and 8. The hook structure 16 further comprises a rear buckle or loop part 17 that is configured for receiving and fixing a strap of the headgear. Such a hook structure 16 allows an easy connection of the headgear to the mask body 1, and a quick release of it, in case of emergency for instance.

In other words, several straps of the headgear are fixed to the slots 10 arranged on the holding arm 3 and to the slot 11 and the hollow connecting structure 14 of the lateral arms 2a, 2b in such a way that a headgear comprising straps can be connected to the mask body 1, thereby maintaining the mask in a desired position on the head of the patient during its use and thus obtaining an efficient treatment of respiratory disorders.

The shell or mask body 1 is fluidly linked to a gas supply line, such as a flexible hose, by means of the tubular hollow connector 12, which delivers a respiratory gas, such as air under pressure, into the breathing chamber 6 of the mask body 1. The gas supply line is fed with gas under pressure by a respiratory device or ventilator (not shown).

The tubular hollow connector 12 is fixed to the mask body 1 by means of an annular element 9, such a polymer (plastic) ring or similar structure, that is arranged in the inlet orifice 4 of the mask body 1 that is located on the front side of the mask body.

The nasal respiratory mask of the present invention can be used in a method for treatment of a respiratory disorder or condition, especially affecting an infant or child patient, for example, in non-invasive positive pressure ventilation (NPPV) or in a nasal continuous positive airway pressure (N-CPAP) therapy of sleep disordered breathing (SDB) conditions, such as, for example, obstructive sleep apnea (OSA).

## Claims

1. Respiratory mask comprising :
- a mask body (1) comprising an internal chamber (6) and an inlet orifice (4) in fluid communication with the internal chamber (6),
- a holding arm (3) arranged on the mask body (1) and projecting upwardly from said mask body (1),
- a cushion (5) having an aperture (7) adapted for receiving at least part of the patient's nose, when the patient wears the mask, and
- two lateral arms (2a, 2b) arranged on each side of the mask body (1) and projecting laterally from said mask body (1),
**characterized in that** the mask body (1), the holding arm (3), the cushion (5) and the two lateral arms (2a, 2b) are integral and form a single piece made of a resilient soft material.

2. Respiratory mask according to Claim 1, **characterized in that** the mask body (1), the holding arm (3), the cushion (5) and the two lateral arms (2a, 2b) are molded in one piece.

3. Respiratory mask according to any one of the preceding Claim, **characterized in that** the resilient soft material forming the unique piece is silicone.

4. Respiratory mask according to any one of the preceding Claims, **characterized in that** the cushion (5) comprises at least one flexible membrane (8), said membrane (8) being made of said resilient soft material and integral with the cushion (5).

5. Respiratory mask according to any one of the preceding Claims, **characterized in that** an annular element (9) is arranged in the inlet orifice (4) of the mask body (1), preferably the annular element (9) is made of a rigid polymer, such as plastic.

6. Respiratory mask according to any one of the preceding Claims, **characterized in that** the holding arm (3) projecting upwardly from said mask body (1) comprises at least one slot (10) at its terminal end (3a).

7. Respiratory mask according to any one of the preceding Claims, **characterized in that** at least one of the two opposite lateral arms (2a, 2b) projecting laterally from said mask body (1) comprises a slot (11).

8. Respiratory mask according to any one of the preceding Claims, **characterized in that** it further comprises a headgear and connecting elements (19, 20) for fixing the headgear to the mask body (1).

9. Respiratory mask according to any one of the previous Claims, **characterized in that** the connecting elements comprise one or several straps, the one or several straps of the headgear cooperating with one of the slots (10, 11) arranged at the terminal end (3a) of the holding arm (3) and/or in at least one of the two lateral arms (2a, 2b).

10. Respiratory mask according to any one of the preceding Claims, **characterized in that** the mask body (1) has a general triangular tridimensional shape.

11. Respiratory respiratory mask according to any one of the preceding Claims, **characterized in that** the inlet orifice (4) is arranged at the center of the mask body (1).

12. Respiratory mask according to any one of the preceding Claims, **characterized in that** at least one (2a) of the two opposite lateral arms (2a, 2b) projecting laterally from said mask body (1) comprises a hollow connecting structure (14) having a tridimensional shape projecting away on the front surface of said lateral arm (2a) and further comprising an inner passage (15) configured for receiving the hook (18) of a hook structure (16) that further comprises a rear buckle or loop (17) for fixing thereto the strap of a headgear.

13. Respiratory mask according to any one of the preceding Claims, **characterized in that** the two opposite lateral arms (2a, 2b) have a generally curved-shape.

14. Respiratory mask according to any one of the preceding Claims, **characterized in that** the respiratory mask is a nasal mask, preferably a pediatric nasal mask dimensioned for fitting to the nasal region of an infant or a child.

15. Assembly comprising a gas delivery device and a respiratory mask according to any one of the preceding Claims, preferably the gas delivery device is connected to the respiratory mask by means of a gas line, such as a flexible hose.
